# EUROPÄISCHE PATENTSCHRIFT

(11) **EP 1 910 354 B1**
(45) Veröffentlichungstag und Bekanntmachung des Hinweises auf die Patenterteilung: **09.11.2016**
(21) Anmeldenummer: 06777919.9
(22) Anmeldetag: 24.07.2006
(51) Int. Cl.: C07D 451/10, C07D 451/06, A61K 31/46, A61P 11/00

(54) **NEUES VERFAHREN ZUR HERSTELLUNG VON TIOTROPIUMSALZEN**
NOVEL METHOD FOR PRODUCING TIOTROPIUM SALTS
NOUVEAU PROCEDE DE PRODUCTION DE SELS DE TIOTROPIUM

(30) Priorität: 27.07.2005 DE 102005035112
(43) Veröffentlichungstag der Anmeldung: 16.04.2008
(73) Patentinhaber: Boehringer Ingelheim Pharma GmbH & Co. KG, 55216 Ingelheim am Rhein (DE)
(72) Erfinder: BRANDENBURG, Joerg, 65199 Wiesbaden (DE); PFRENGLE, Waldemar, 88400 Biberach (DE)
(74) Vertreter: Simon, Elke Anna Maria
(86) Internationale Anmeldenummer: PCT/EP2006/064559
(87) Internationale Veröffentlichungsnummer: WO 2007/012626

(56) Entgegenhaltungen:
- EP-A- 1 504 756
- WO-A-03/057694
- WO-A-2005/042526
- WO-A2-02/096423
- WO-A2-2006/021559
- US-A1- 2005 096 341

## Beschreibung

Die Erfindung betrifft ein neues Verfahren zur Herstellung von Tiotropiumsalzen der allgemeinen Formel 1 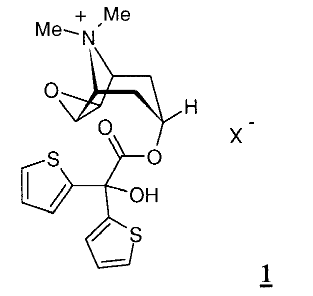 worin X ⁻die in den Ansprüchen und in der Beschreibung genannten Bedeutungen haben kann.

### Hintergrund der Erfindung

Anticholinergika können bei einer Vielzahl von Erkrankungen therapeutisch sinnvoll eingesetzt werden. Hervorzuheben sind hier beispielsweise die Therapie von Asthma oder COPD (chronic obstructive pulmonary disease = chronisch obstruktive Lungenerkrankung). Zur Therapie dieser Erkrankungen werden beispielsweise durch die WO 02/03289 Anticholinergika vorgeschlagen, die ein Scopin-, Tropenol- oder auch Tropin-Grundgerüst aufweisen. Darüberhinaus wird im Stand der Technik insbesondere das Tiotropiumbromid als hoch potentes Anticholinergikum offenbart. Tiotropiumbromid ist beispielsweise aus der EP 418 716 A1 bekannt.

Neben den im vorstehend genannten Stand der Technik offenbarten Syntheseverfahren zur Herstellung von Scopinestern wird insbesondere in der WO03/057694 ein Verfahren zur Herstellung von Estern des Scopins offenbart.

Aufgabe der vorliegenden Erfindung ist es, ein verbessertes technisches Syntheseverfahren bereitzustellen, welches einen einfacheren synthetischen Zugang zu den Verbindungen der allgemeinen Formel **1** in einer gegenüber dem Stand der Technik verbesserten Art und Weise ermöglicht.

### Detaillierte Beschreibung der Erfindung

Die vorliegende Erfindung betrifft ein Verfahren zur Herstellung von Tiotropiumsalzen der Formel 1 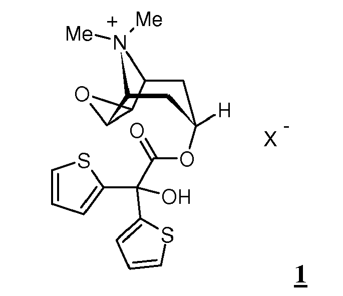 worin
- X -: ein einfach negativ geladenes Anion, vorzugsweise ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat, p-Toluolsulfonat und Trifluormethansulfonat, bedeuten kann,
dadurch gekennzeichnet, daß eine Verbindung der Formel **2** 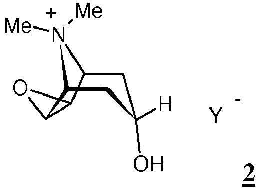 worin
- Y ⁻: ein einfach negativ geladenes lipophiles Anion ausgewählt aus der Gruppe bestehend aus Hexfluorophosphat, Tetrafluoroborat, und Tetraphenylborat, besonders bevorzugt Hexfluorophosphat oder Tetraphenylborat
bedeuten kann, in einem Schritt mit einer Verbindung der Formel **3** 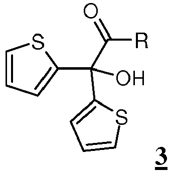 worin
- R: ein Rest ausgewählt aus der Gruppe bestehend aus Methoxy, Ethoxy, Propoxy, Isopropoxy, Isopropenyloxy, Butoxy, O-N-Succinimid, O-N-Phtalimid, Phenyloxy, Nitrophenyloxy, Fluorophenyloxy, Pentafluorophenyloxy, Vinyloxy, 2-Allyloxy, -S-Methyl, -S-Ethyl und -S-Phenyl bedeutet,
in einem geeigneten Lösemittel unter Zusatz einer geeigneten Base und eines Katalysators ausgewählt aus der Gruppe bestehend aus Zeolithe , Alkoholate, Lipasen und tert. Amine, zu einer Verbindung der Formel **4** 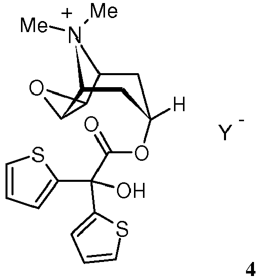 umgesetzt werden, wobei die Gruppe Y⁻ die vorstehend genannten Bedeutungen haben kann, und die Verbindung der Formel **4** ohne Isolierung durch Umsetzung mit einem Salz Kat⁺X⁻, wobei Kat⁺ für ein Kation ausgewählt aus der Gruppe bestehend aus Li⁺, Na⁺, K⁺, Mg2+, Ca2+, organische Kationen mit quartärem N (z.B. N,N-Dialkylimidazolium, Tetraalkylammonium ) steht und X- die vorstehend genannten Bedeutungen haben kann, in die Verbindung der Formel **1** überführt wird.

Bevorzugt betrifft die vorliegende Erfindung ein Verfahren zur Herstellung von Tiotropiumsalzen der Formel **1**, worin
- X⁻: ein einfach negativ geladenes Anion ausgewählt aus der Gruppe bestehend aus Chlorid, Bromid, Iodid, Methansulfonat, p-Toluolsulfonat und Trifluormethansulfonat, bevorzugt Chlorid, Bromid, Iodid, Methansulfonat oder Trifluormethansulfonat, besonders bevorzugt Chlorid, Bromid oder Methansulfonat, besonders bevorzugt Bromid, bedeuten kann.

Ein erfindungsgemäß besonders bevorzugtes Verfahren ist dadurch gekennzeichnet, dass die Umsetzung mit einer Verbindung der Formel **3** erfolgt, in der
- R: ein Rest ausgewählt aus der Gruppe bestehend aus Methoxy, Ethoxy, Propoxy, Isopropoxy, Isopropenyloxy, Butoxy, O-N-Succinimid, O-N-Phtalimid, Phenyloxy, Nitrophenyloxy, Fluorophenyloxy, Pentafluorophenyloxy, Vinyloxy und 2-Allyloxy bedeutet.

Ein erfindungsgemäß besonders bevorzugtes Verfahren ist dadurch gekennzeichnet, dass die Umsetzung mit einer Verbindung der Formel **3** erfolgt, in der R ein Rest ausgewählt aus der Gruppe bestehend aus Methoxy, Ethoxy, Propoxy, Isopropoxy, Isopropenyloxy, Butoxy, O-N-Succinimid, O-N-Phtalimid, Vinyloxy und 2-Allyloxy, bevorzugt ausgewählt aus Methoxy, Ethoxy, Propoxy, und Butoxy, besonders bevorzugt Methoxy oder Ethoxy bedeutet.

Ein erfindungsgemäß besonders bevorzugtes Verfahren ist dadurch gekennzeichnet, dass die Umsetzung mit einer Verbindung der Formel **2** erfolgt, in der
- Y⁻: ein einfach negativ geladenes Anion ausgewählt aus der Gruppe bestehend aus Hexfluorophosphat, Tetrafluoroborat und Tetraphenylborat, bevorzugt Hexafluorophosphat.

Ein erfindungsgemäß besonders bevorzugtes Verfahren ist dadurch gekennzeichnet, dass die abschließende Umsetzung der Verbindung der Formel **4** zur Verbindung der Formel **1** mit Hilfe eines Salzes KatX erfolgt, in dem Kat⁺ ausgewählt ist aus der Gruppe bestehend aus Li⁺, Na⁺, K⁺, Mg2+, Ca2+, organische Kationen mit quartärem N (z.B. N,N-Dialkylimidazolium, Tetraalkylammonium ) und in der X- die vorstehend genannten Bedeutungen haben kann.

Als Alkylgruppen sowie Alkylgruppen, welche Bestandteil anderer Reste sind, werden verzweigte und unverzweigte Alkylgruppen mit 1 bis 4 Kohlenstoffatomen bezeichnet. Beispielsweise werden genannt: Methyl, Ethyl, Propyl, Butyl. Sofern nicht anders genannt, sind von den vorstehend genannten Bezeichnungen Propyl, Butyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl.

Als Alkoxy- oder Alkyloxygruppen, werden verzweigte und unverzweigte Alkylgruppen mit 1 bis 4 Kohlenstoffatomen bezeichnet, die über ein Sauerstoffatom verknüpft sind. Beispielsweise werden genannt: Methoxy, Ethoxy, Propoxy, Butoxy. Sofern nicht anders genannt, sind von den vorstehend genannten Bezeichnungen sämtliche der möglichen isomeren Formen umfaßt.

Die Bezeichnungen Phenyl-Methyl und Phenyl-NO₂ stehen für Phenylringe, die durch Methyl oder NO₂ substituiert sind. Hierbei sind alle möglichen Isomeren (ortho, meta oder para) umfasst, wobei der para- oder meta-Substitution besondere Bedeutung zukommt.

Als Cycloalkylgruppen werden Cycloalkylreste mit 3 - 6 Kohlenstoffatomen beispielsweise Cyclopropyl, Cyclobutyl, Cyclopentyl oder Cyclohexyl bezeichnet.

Unter lipophilen Anionen werden in diesem Fall erfindungsgemäß solche Anionen verstanden, deren Natrium- oder Kaliumsalze eine Löslichkeit in polaren organischen Lösungsmitteln wie Methanol oder Aceton von > 1 Gew.-% aufweisen.

Das erfindungsgemäße Verfahren zeichnet sich insbesondere dadurch aus, dass es aufgrund der Löslichkeit der Ausgangsverbindungen der Formel **2** und der Intermediate der Formel **4** in relativ unpolaren Lösemitteln geführt werden kann. Dies erlaubt eine Umsetzung unter recht schonenden Bedingungen , die im Vergleich zu Reaktionen in hochpolaren aprotischen Lösungsmitteln bei den empfindlichen Tiotropiumsalzen weniger Nebenreaktionen und damit zusammenhängend eine höhere Ausbeute zur Folge haben. Die Umsetzung der Verbindungen der Formel **2** mit den Verbindungen der Formel **3** wird vorzugsweise in einem aprotischen organischen Lösemittel, bevorzugt in einem schwach polaren organischen Lösemittel durchgeführt. Als Lösemittel kommen erfindungsgemäß besonderes bevorzugt in Betracht Aceton, Pyridin, Acetonitril und Methylethylketon, wobei Aceton, Acetonitril und Pyridin bevorzugt zum Einsatz gelangen. Besonders bevorzugt erfolgt die Umsetzung in einem Lösemittel ausgewählt aus der Gruppe bestehend aus Aceton und Acetonitril wobei die Verwendung von Aceton erfindungsgemäß besonders bevorzugt ist.

Gegebenenfalls kann es sinnvoll sein, die Umsetzung der Verbindung der Formel **2 mit 3** durch Zusatz eines Katalysators zu aktivieren. Besonders schonende Aktivierung ist erfindungsgemäß möglich durch Katalysatoren, die ausgewählt sind aus der Gruppe bestehend aus Zeolithe , Lipasen, tert. Amine, wie beispielsweise N,N-Dialkylaminopyridin, 1,4-Diazabicyclo[2.2.2]octan (DABCO) und Diisopropylethylamin und Alkoholaten, wie beispielsweise, wobei die Verwendung von Zeolithen und insbesondere Zeolithen und Kalium-tert.-butylat, erfindungsgemäß besonders bevorzugt ist. Besonders bevorzugte Zeolithe sind Molekularsiebe die ausgewählt sind aus der Gruppe der Molekularsieben mit basischem Charakter bestehend aus Natrium-oder Kalium-haltigen Alumosilikaten, bevorzugt Molsiebe mit der Summenformel Na₁₂[(AlO₂)₁₂(SiO₂)₁₂] x H₂O, wobei die Verwendung von Molsieb Typ 4A (steht für Porengröße von 4 Angström) erfindungsgemäß besonders bevorzugt ist.

Die Umsetzung von **2** mit **3** zur Verbindung der Formel **4** kann , abhängig vom Katalysator-Typ bei erhöhter Temperatur erfolgen. Bevorzugt erfolgt die Umsetzung bei einer Temperatur von 30°C, besonders bevorzugt in einem Bereich von 0 bis 30°C.

Die Verbindungen der Formel **3** können nach im Stand der Technik bekannten Verfahren erhalten werden. Hierbei sei beispielsweise auf die WO03/057694 verwiesen.

Die Verbindungen der Formel **2** sind für das erfindungsgemäße Verfahren von zentraler Bedeutung. Dementsprechend betrifft ein weitere Aspekt der vorliegenden Erfindung Verbindungen der Formel **2** 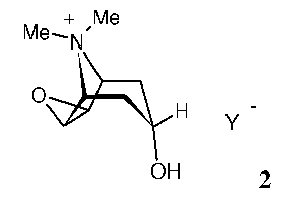 als solche, worin
- Y⁻: ein einfach negativ geladenes lipophiles Anion ausgewählt aus der Gruppe bestehend aus Hexfluorophosphat, Tetrafluoroborat und Tetraphenylborat, besonders bevorzugt Hexfluorophosphat oder Tetraphenylborat

Zur Herstellung der Verbindungen der Formel **2** kann wie folgt vorgegangen werden. Bevorzugt wird hierzu ein Scopinsalz der Formel **5,** 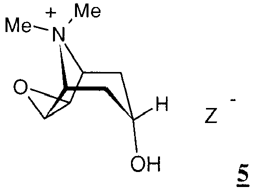 worin Z⁻ ein von Y verschiedenes einfach negativ geladenes Anion bedeutet, in einem geeigneten Lösemittel, bevorzugt in einem polaren Lösemittel, besonders bevorzugt in einem Lösemittel ausgewählt aus der Gruppe bestehend aus Wasser, Methanol, Ethanol, Propanol oder Isopropanol gelöst. Erfindungsgemäß bevorzugt sind als Lösemittel Wasser und Methanol wobei Wasser erfindungsgemäß herausragende Bedeutung zukommt.

Besonders bevorzugt werden als Ausgangsverbindung zur Herstellung der Verbindung der Formel **2** solche Verbindungen der Formel **5** verwendet, bei denen
- Z⁻: ein einfach negativ geladenes Anion, vorzugsweise ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat bedeutet.

Ferner bevorzugt werden als Ausgangsverbindung zur Herstellung der Verbindung der Formel **2** solche Verbindungen der Formel **5** verwendet, bei denen
- Z⁻: ein einfach negativ geladenes Anion ausgewählt aus der Gruppe bestehend aus Chlorid, Bromid, 4-Toluolsulfonat und Methansulfonat, bevorzugt Bromid bedeutet.

Die so erhaltene Lösung wird mit einem Salz Kat'Y versetzt. Hierbei steht Y für eines der vorstehend genannten Anionen, während Kat' für ein Kation steht, welches bevorzugt ausgewählt ist aus der Gruppe bestehend aus Protonen (H⁺), Alkali- oder Erdalkalimetallkationen, Ammonium, bevorzugt Protonen oder Alkalimetallkationen, besonders bevorzugt Li⁺-, Na⁺- und K⁺-ionen.

Pro Mol eingesetzte Verbindung der Formel **5** werden erfindungsgemäß bevorzugt 1 Mol , bevorzugt 1-1.5 Mol, gegebenenfalls auch 2-5 Mol des Salzes Kat'Y eingesetzt. Für den Fachmann ist ersichtlich, dass der Einsatz geringerer Mengen an Salz Kat'Y möglich ist, dass dies aber dann nur zu einer partiellen Umsetzung der Verbindung der Formel **5** führen kann.

Die erhaltene Lösung wird bis zur vollständigen Umsetzung gerührt. Hierbei kann bei Raumtemperatur (ca 23°C) oder gegebenenfalls auch bei leicht erhöhter Temperatur in einem Bereich von 25-50°C gearbeitet werden. Nach vollständiger Zugabe, teilweise auch schon während der Zugabe, kristallisieren die Verbindungen der Formel **2** aus der Lösung aus. Die erhaltenen Produkte können, falls erforderlich, durch Umkristallisation aus einem der vorstehend genannten Lösemittel gereinigt werden. Die erhaltenen Kristalle werden isoliert und im Vakuum getrocknet.

Ein weiterer Aspekt der vorliegenden Erfindung betrifft die Verwendung von Verbindungen der Formel **2** als Ausgangsverbindungen zur Herstellung von Verbindungen der Formel **1.** Ein weiterer Aspekt der vorliegenden Erfindung betrifft die Verwendung von Verbindungen der Formel **2** als Ausgangsverbindungen zur Herstellung von Verbindungen der Formel **4**. Ein weiterer Aspekt der vorliegenden Erfindung betrifft die Verwendung von Verbindungen der Formel **5** als Ausgangsverbindungen zur Herstellung von Verbindungen der Formel **2.** Ein weiterer Aspekt der vorliegenden Erfindung betrifft die Verwendung von Verbindungen der Formel **5** als Ausgangsverbindungen zur Herstellung von Verbindungen der Formel **4**.

Ein weiterer Aspekt der vorliegenden Erfindung betrifft ein Verfahren zur Herstellung von Verbindungen der Formel **1**, dadurch gekennzeichnet, dass eine Verbindung der Formel **2** als Ausgangsverbindung zur Herstellung von Verbindungen der Formel **1** eingesetzt wird. Ein weiterer Aspekt der vorliegenden Erfindung betrifft ein Verfahren zur Herstellung von Verbindungen der Formel **4**, dadurch gekennzeichnet, dass eine Verbindung der Formel **2** als Ausgangsverbindung zur Herstellung von Verbindungen der Formel **4** eingesetzt wird. Ein weiterer Aspekt der vorliegenden Erfindung betrifft ein Verfahren zur Herstellung von Verbindungen der Formel **2**, dadurch gekennzeichnet, dass eine Verbindung der Formel **5** als Ausgangsverbindung zur Herstellung von Verbindungen der Formel **2** eingesetzt wird. Ein weiterer Aspekt der vorliegenden Erfindung betrifft ein Verfahren zur Herstellung von Verbindungen der Formel **4**, dadurch gekennzeichnet, dass eine Verbindung der Formel **5** als Ausgangsverbindung zur Herstellung von Verbindungen der Formel **4** eingesetzt wird.

Die Verbindungen der Formel **4** sind für das erfindungsgemäße Verfahren von zentraler Bedeutung. Dementsprechend betrifft ein weitere Aspekt der vorliegenden Erfindung Verbindungen der Formel **4** 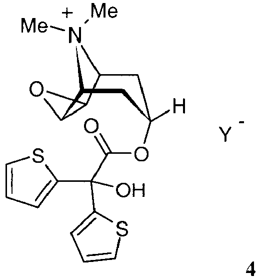 als solche, wobei die Gruppe Y⁻ die vorstehend genannten Bedeutungen haben kann.

Ein weiterer Aspekt der vorliegenden Erfindung betrifft die Verwendung von Verbindungen der Formel **4** als Ausgangsverbindungen zur Herstellung von Verbindungen der Formel **1**. Ein weiterer Aspekt der vorliegenden Erfindung betrifft ein Verfahren zur Herstellung von Verbindungen der Formel **1**, dadurch gekennzeichnet, dass eine Verbindung der Formel **4** als Ausgangsverbindung zur Herstellung von Verbindungen der Formel **1** eingesetzt wird.

Die Verbindungen der Formel **4** werden wie vorstehend beschrieben im Rahmen des erfindungsgemäßen Verfahrens zur Herstellung von Verbindungen der Formel **1** als Intermediate erhalten. Im Rahmen des erfindungsgemäßen Verfahrens zur Herstellung von Verbindungen der Formel **1** muss hierbei in einer bevorzugten Ausführungsform der Erfindung, die Verbindung der Formel **4** nicht isoliert werden.

Die nachfolgenden Beispiele dienen der Illustration exemplarisch durchgeführter Syntheseverfahren. Sie sind lediglich als mögliche, exemplarisch dargestellte Vorgehensweisen zu verstehen, ohne die Erfindung auf deren Inhalt zu beschränken Nur Beispiele, welche unter den Umfang der Ansprüche fallen sind erfindungsgemäss.

### Beispiel 1: N-Methylscovinium hexafluorophosphat

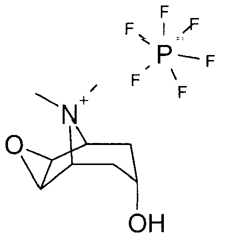

N-Methylscopiniumbromid wird in Wasser gelöst und mit einer equimolaren oder molar überschüssigen Menge eines in Wasser löslichen Hexafluorophosphates (Natrium- oder Kaliumsalz) versetzt. (Auch wässrige Hexafluorophosphorsäure führt zur Ausfällung). Das N-Methylscopinium-hexafluorophosphat fällt / kristallisiert als weißes, wasserunlösliches Produkt aus, wird isoliert, ggf. mit Methanol gewaschen und dann bei ca. 40°C im Trockenschrank getrocknet.
Schmp.: 265-267°C (Schmelze unter Verfärbung);
H-NMR: in Acetonitril-d3 σ(ppm): 1,9 (dd, 2H) , 2,55( dd, 2H), 2,9 (s,3H), 3,29 (s,3H), 3,95(dd, 4H), 3,85 (s, 1H).

### Beispiel 2: Tiotropiumbromid

1,6 g (5mmol) Methylscopinium Hexafluorophoshat (Beispiel 1) und 2,0 g (7,8 mmol) Dithienylglycolsäuremethylester werden in 50 ml Aceton und in Gegenward von 10g Molsieb 4A 50-70 Stunden unter Rückfluß gekocht.
Das Reaktionsgemisch wird filtriert, das Filtrat mit einer Lösung aus 0,3 g LiBr in 10 ml Aceton versetzt. Das auskristallisierte noch nicht umgesetzte N-Methylscopiniumbromid wird durch Filtration abgetrennt. Nach Zugabe von weiteren 0,6 g LiBr (gelöst in Aceton) fällt Tiotropiumbromid in einer isolierten Ausbeute von 30% (bezogen auf eingesetzte Verbindung nach Beispiel 1) aus.

### Beispiel 3: Tiotropiumhexafluorophosphat

Tiotropiumhexafluorophosphat wird im Rahmen der Umsetzung nach Beispiel 2 nicht isoliert sondern direkt weiter zum Tiotropiumbromid umgesetzt.
Für die Zwecke der Charakterisierung des Tiotropiumhexafluorophosphat wurde dieses spezifisch hergestellt und isoliert. Hierbei wurden die folgenden charakterisierenden Daten erhalten. Schmp.: 233-236°C (Schmelzen unter Verfärbung)

H-NMR: in aceton-d6 : σ(ppm): 2,08 (dd, 2H), 2,23( dd, 2H), 3,32 (s,3H), 3,50 (s,3H), 3,62(s,2H), 4,28(m, 2H), 5,39(m, 1H) ,6,25 (s), 7,02(m,2H), 7,027,22(m,2H), 7,46(m,2H), P-NMR: in aceton-d6 : σ(ppm): -143,04, Heptett, J =4,37.

### Beispiel 4: Tiotropiumbromid

31,5 g (100mmol) Methylscopinium Hexafluorophoshat (Beispiel 1) und 25,4 g (100 mmol) Dithienylglycolsäuremethylester werden in 400 ml Aceton und in Gegenwart von

40g Molsieb 4A, Pulver (Fluka) und DMAP (4,4-Dimethylaminopyridin) 24h unter Rückfluß gekocht. (Molsieb wurde nach 3h in gleicher Menge ausgetauscht.)
Das Reaktionsgemisch wird filtriert, mit 200ml Aceton nachgewaschen, das Filtrat schrittweise mit einer Lösung aus 9,6 g LiBr (110mmol) in 110 ml Aceton versetzt. Das auskristallisierte noch nicht umgesetzte N-Methylscopiniumbromid wird durch Filtration abgetrennt. (Fraktionierte Fällung). Die Kristallfraktionen wurden abfiltriert und getrocknet. Die Zusammensetzung der Fraktionen wurde dünnschichtchromatographisch bestimmt. Tiotropiumbromid in einer isolierten Ausbeute von 16,6g (35%) (bezogen auf eingesetzte Verbindung nach Beispiel 1) aus. Reinheit HPLC> 99%. Reinheit DC: keine Verunreinigungen erkennbar.

### Beispiel 5: Tiotropiumbromid

1,6 g (5 mmol) Methylscopinium Hexafluorophoshat (Beispiel 1) und 1,25 g (5 mmol) Dithienylglycolsäuremethylester werden in 50 ml Aceton und in Gegenwart von 2g Molsieb 4A, Pulver (Fluka) und 6mg Kalium-tert.-butylat bei 0°C 4 h lang gerührt.
Das Reaktionsgemisch wird filtriert, mit 20ml Aceton nachgewaschen, das Filtrat schrittweise mit einer Lösung aus 0,7 g LiBr (13mmol) in 11 ml Aceton versetzt. Das auskristallisierte noch nicht umgesetzte wird durch Filtration abgetrennt. (Fraktionierte Fällung). Die Kristallfraktionen wurden abfiltriert und getrocknet. Die Zusammensetzung der Fraktionen wurde dünnschichtchromatographisch bestimmt. Die Tiotropiumbromid-Fraktionen wurden abgesaugt, mit Aceton nachgewaschen, aus Wasser umkristallisiert, mit Aceton nachgewaschen und getrocknet. 1,2g (48% Ausbeute bezogen auf eingesetzte Verbindung nach Beispiel 1) Tiotropiumbromid wurden auf diese Weise isoliert.
Reinheit HPLC: 99,8% , DC: keine Verunreinigung sichtbar

### Beispiel 6: Tiotropiumbromid

31,5g (0,1 mol) Methylscopinium Hexafluorophoshat (Beispiel 1) und 30,5g (0,10mol) 2,2'-Dithienylglycolsäuremethylester werden in 400 ml Aceton gelöst und in Gegenwart von 90g Zeolith vom Typ 4A (Na₁₂A1₁₂Si₁₂O₄₈ x n H₂O) und 0,2g (1mmol) Kalium-tert.-butylat über einen Zeitraum von 20-24 Stunden bei 0°C gerührt.
Das Reaktionsgemisch wird filtriert, das Filtrat mit einer Lösung aus 8,7 g LiBr (8,7 g 0,10mol in 100 ml Aceton) versetzt.
Das dabei auskristallisierte Produkt wird durch Filtration abgetrennt, mit Aceton gewaschen und dann getrocknet.
Man erhält 41,4 g (87,7%) Ausbeute, bei 90% Umsetzungsgrad.

### Beispiel 7: N-Methylscopinium Tetraphenylborat

20g (80 mmol) Methylscopinium Bromid werden in 500 ml Methanol gelöst. 27,38 (80mmol) Natriumtetraphenylborat, gelöst in 150 ml Methanol werden zudosiert. Die enstandene Suspension wird 10 min bei Raumtemperatur nachgerührt und filtriert. Die abgetrennten Kristalle werden mit 50 ml Methanol gewaschen und getrocknet. Ausbeute: 39,1g (91,73% Ausbeute); Schmp.: 261°C.

### Beispiel 8: Tiotropium Tetraphenylborat

0,245 g ( 0,5 mmol ) Methylscopinium Tetraphenylborat (Beispiel 7), und 0,154 g (0,6 mmol) 2,2- Dithienylglycolsäuremethylester werden in 25 ml Aceton gelöst und in Gegenwart von 1,0 g Zeolith vom Typ 4A (Na₁₂Al₁₂Si₁₂O₄₈ x n H₂O) und 5 mg Kalium-tert.-butylat über einen Zeitraum von 20-30 Stunden bei 0°C gerührt. Laut HPLC sind 79% des umgesetzten 2,2- Dithienylglycolsäuremethylesters nach 26 h in Tiotropiumtetraphenylborat verwandelt. (nicht isolierte Ausbeute: 43%).

Die beispielhaft aufgeführten Reaktionen erfolgen praktisch ohne Bildung von Nebenprodukten.. Sollten die Reaktionen gegebenenfalls ohne vollständige Umsetzung der Ausgangsmaterialien erfolgen, kann das im ersten Schritt der Aufarbeitung isolierte N-Methylscopiniumbromid daher mit der Reaktion nach Beispiel 1 zurückgeführt und damit die Gesamtausbeute im Rahmen eines Herstellverfahrens signifikant erhöht werden werden.

## Patentansprüche

1. Verfahren zur Herstellung von Tiotropiumsalzen der Formel 1 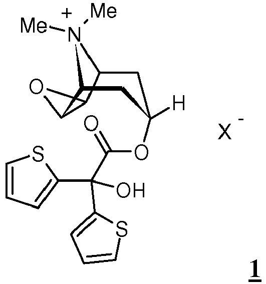 worin
X⁻ ein einfach negativ geladenes Anion bedeuten kann,
**dadurch gekennzeichnet, daß** eine Verbindung der Formel 2 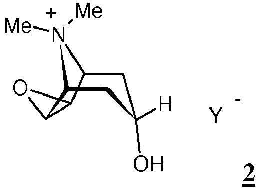 worin
Y⁻ ein von X- verschiedenes einfach negativ geladenes lipophiles Anion ausgewählt aus der Gruppe bestehend aus Hexfluorophosphat, Tetrafluoroborat und Tetraphenylborat, besonders bevorzugt Hexfluorophosphat oder Tetraphenylborat
bedeuten kann, in einem Schritt mit einer Verbindung der Formel 3 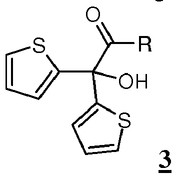 worin
R ein Rest ausgewählt aus der Gruppe bestehend aus Methoxy, Ethoxy, Propoxy, Isopropoxy, Isopropenyloxy, Butoxy, O-N-Succinimid, O-N-Phtalimid, Phenyloxy, Nitrophenyloxy, Fluorophenyloxy, Pentafluorophenyloxy, Vinyloxy, 2-Allyloxy, -S-Methyl, -S-Ethyl und -S-Phenyl bedeutet,
in einem geeigneten Lösemittel unter Zusatz einer geeigneten Base und eines Katalysator ausgewählt aus der Gruppe bestehend aus Zeolithe , Alkoholate, Lipasen und tertiäre Amine zu einer Verbindung der Formel **4** 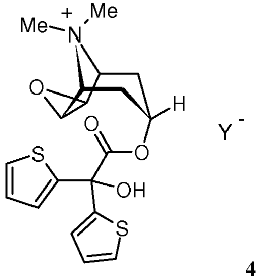 umgesetzt wird, wobei die Gruppe Y⁻ die vorstehend genannten Bedeutungen haben kann, und die Verbindung der Formel **4** ohne Isolierung durch Umsetzung mit einem Salz Kat⁺X⁻, wobei Kat⁺ für ein Kation ausgewählt aus der Gruppe bestehend aus Li⁺, Na⁺, K⁺, Mg2+, Ca2+ und organische Kationen mit quartärem N steht und X- die vorstehend genannten Bedeutungen haben kann, in die Verbindung der Formel 1 überführt wird.

2. Verfahren nach Anspruch 1, worin
X⁻ ein einfach negativ geladenes Anion ausgewählt aus der Gruppe bestehend aus Chlorid, Bromid, Iodid, Methansulfonat, p-Toluolsulfonat und Trifluormethansulfonat, bevorzugt Chlorid, Bromid oder Methansulfonat, besonders bevorzugt Bromid, bedeuten kann.

3. Verfahren nach Anspruch 1 oder 2, wobei die Umsetzung mit einer Verbindung der Formel **3** erfolgt, in der
R ein Rest ausgewählt aus der Gruppe bestehend aus Methoxy, Ethoxy, Propoxy, Isopropoxy, Isopropenyloxy, Butoxy, O-N-Succinimid, O-N-Phtalimid, Phenyloxy, Nitrophenyloxy, Fluorophenyloxy, Pentafluorophenyloxy, Vinyloxy und 2-Allyloxy sein kann.

4. Verfahren nach Anspruch 1, 2 oder 3, wobei die Umsetzung mit einer Verbindung der Formel **2** erfolgt, in der
Y⁻ Hexfluorophosphat sein kann.

5. Verfahren nach einem der Ansprüche 1 bis 4 , **dadurch gekennzeichnet,**
**dass** die abschließende Umsetzung der Verbindung der Formel **4** zur Verbindung der Formel **1** mit Hilfe eines Salzes KatX erfolgt, in dem Kat⁺ ausgewählt ist aus der Gruppe bestehend aus Li⁺, Na⁺, K⁺, Mg2+, Ca2+, organische Kationen mit quartärem N (z.B. N,N-Dialkylimidazolium, Tetraalkylammonium ) und in der X- die vorstehend genannten Bedeutungen haben kann.

6. Verbindungen der Formel 2 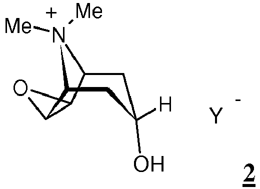 worin
Y⁻ ein einfach negativ geladenes lipophiles Anion ausgewählt aus der Gruppe bestehend aus Hexfluorophosphat, Tetrafluoroborat und Tetraphenylborat bedeutet.

7. Verwendung von Verbindungen der Formel **2** nach Anspruch 6 als Ausgangsverbindungen zur Herstellung von Verbindungen der Formel **1**.

8. Verfahren zur Herstellung von Verbindungen der Formel **1**, **dadurch gekennzeichnet, dass** eine Verbindung der Formel **2** nach Anspruch 6 als Ausgangsverbindung zur Herstellung von Verbindungen der Formel **1** eingesetzt wird.

9. Verbindungen der Formel **4** 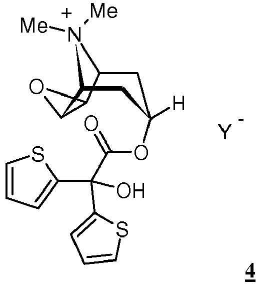 worin
Y⁻ ein einfach negativ geladenes lipophiles Anion ausgewählt aus der Gruppe bestehend aus Hexfluorophosphat, Tetrafluoroborat und Tetraphenylborat bedeutet.

10. Verwendung von Verbindungen der Formel **4** nach Anspruch 9 als Ausgangsverbindungen zur Herstellung von Verbindungen der Formel **1**.

11. Verfahren zur Herstellung von Verbindungen der Formel **1**, **dadurch gekennzeichnet, dass** eine Verbindung der Formel **4** nach Anspruch 9 als Ausgangsverbindung zur Herstellung von Verbindungen der Formel **1** eingesetzt wird.

## Claims

1. Process for preparing tiotropium salts of formula 1 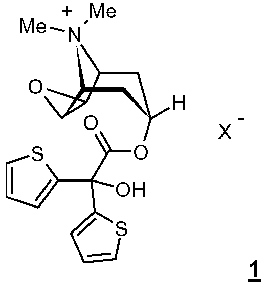 wherein
X⁻ may represent an anion having a single negative charge,
**characterised in that** a compound of formula **2** 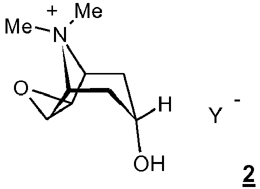 wherein
Y⁻ may represent a lipophilic anion having a single negative charge which is different from X⁻, selected from the group consisting of hexafluorophosphate, tetrafluoroborate and tetraphenylborate, particularly preferably hexafluorophosphate or tetraphenylborate
is reacted in one step with a compound of formula **3** 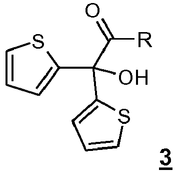 wherein
R denotes a functional group selected from the group consisting of methoxy, ethoxy, propoxy, isopropoxy, isopropenyloxy, butoxy, O-N-succinimide, O-N-phthalimide, phenyloxy, nitrophenyloxy, fluorophenyloxy, pentafluorophenyloxy, vinyloxy, 2-allyloxy, -S-methyl, -S-ethyl and -S-phenyl,
in a suitable solvent with the addition of a suitable base and a catalyst selected from the group consisting of zeolites, alcoholates, lipases and tertiary amines to obtain a compound of formula **4** 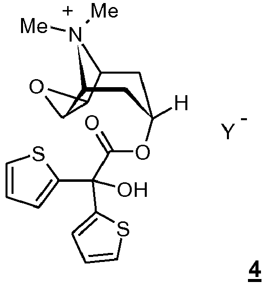 wherein the group Y⁻may have the meanings given above, and without being isolated the compound of formula **4** is converted into the compound of formula **1** by reaction with a salt cat⁺X⁻, wherein cat⁺ denotes a cation selected from the group consisting of Li⁺, Na⁺, K⁺, Mg²⁺, Ca²⁺, and organic cations with quaternary N and X- may have the meanings given above.

2. Process according to claim 1, wherein
X⁻ may represent an anion having a single negative charge selected from the group consisting of chloride, bromide, iodide, methanesulphonate, p-toluenesulphonate and trifluoromethanesulphonate, preferably chloride, bromide or methanesulphonate, particularly preferably bromide.

3. Process according to claim 1 or 2, wherein the reaction is carried out with a compound of formula **3** wherein
R may be a functional group selected from the group consisting of methoxy, ethoxy, propoxy, isopropoxy, isopropenyloxy, butoxy, O-N-succinimide, O-N-phthalimide, phenyloxy, nitrophenyloxy, fluorophenyloxy, pentafluorophenyloxy, vinyloxy and 2-aynoxy.

4. Process according to claim 1, 2 or 3, wherein the reaction is carried out with a compound of formula **2** where
Y⁻ may be hexafluorophosphate.

5. Process according to one of claims 1 to 4, **characterised in that** the final reaction of the compound of formula **4** to obtain the compound of formula **1** is carried out with the aid of a salt catX, where cat⁺ is selected from the group consisting of Li⁺, Na⁺, K⁺, Mg²⁺, Ca²⁺, organic cations with quaternary N (e.g. N,N-dialkylimidazolium, tetraalkylammonium) and where X- may have the meanings given above.

6. Compounds of formula 2 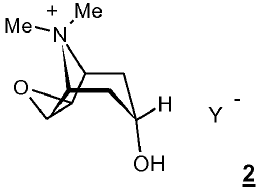 wherein
Y⁻ denotes a lipophilic anion having a single negative charge, selected from the group consisting of hexafluorophosphate, tetrafluoroborate and tetraphenylborate.

7. Use of compounds of formula **2** according to claim 6 as starting compounds for preparing compounds of formula **1.**

8. Process for preparing compounds of formula **1, characterised in that** a compound of formula **2** according to claim 6 is used as a starting compound for preparing compounds of formula **1.**

9. Compounds of formula **4** 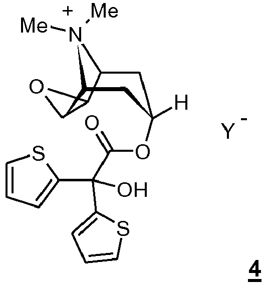 where
Y⁻ denotes a lipophilic anion having a single negative charge, selected from the group consisting of hexafluorophosphate, tetrafluoroborate and tetraphenylborate.

10. Use of compounds of formula **4** according to claim 9 as starting compounds for preparing compounds of formula **1**.

11. Process for preparing compounds of formula **1**, **characterised in that** a compound of formula **4** according to claim 9 is used as a starting compound for preparing compounds of formula **1**.

## Revendications

1. Procédé de production de sels de tiotropium de formule 1 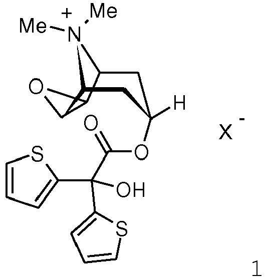 dans laquelle
X⁻ peut représenter un anion à charge simplement négative,
**caractérisé en ce qu'**un composé de formule 2 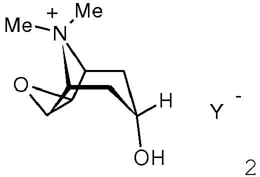 dans laquelle
Y⁻ peut représenter un anion lipophile à charge simplement négative, différent de X⁻, choisi dans le groupe constitué de l'hexafluorophosphate, du tétrafluoroborate, et du tétraphénylborate, de manière particulièrement préférée, de l'hexafluorophosphate ou du tétraphénylborate,
est converti en une étape, avec un composé de formule 3 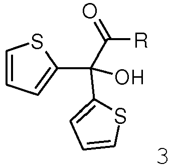 dans laquelle
R représente un radical choisi dans le groupe constitué de méthoxy, éthoxy, propoxy, isopropoxy, isopropényloxy, butoxy, O-N-succinimide, **O-N-**phtalimide, phényloxy, nitrophényloxy, fluorophényloxy, pentafluorophényloxy, vinyloxy, 2-allyloxy, -S-méthyle, -S-éthyle et -S-phényle,
dans un solvant approprié en ajoutant une base appropriée et un catalyseur choisi parmi des zéolites, des alcoolates, des lipases et des amines tertiaires, en un composé de formule 4, 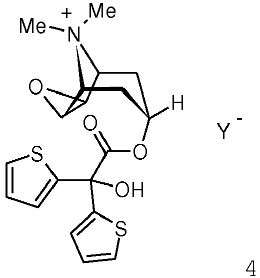 dans laquelle le groupe Y⁻ peut avoir les significations mentionnées précédemment et le composé de formule 4 est converti, sans isolement, par réaction avec un sel Cat⁺X⁻, dans laquelle Cat⁺ est un cation choisi dans le groupe constitué de Li⁺, Na⁺, K⁺, Mg2+, Ca2+ et des cations organiques comportant un N quaternaire et X⁻ peut avoir les significations mentionnées précédemment, en composé de formule 1.

2. Procédé selon la revendication 1, dans lequel
X⁻ peut représenter un anion à charge simplement négative choisi dans le groupe constitué de chlorure, bromure, iodure, méthanesulfonate, p-toluènesulfonate et trifluorométhanesulfonate, de préférence un chlorure, bromure ou méthanesulfonate, de manière particulièrement préférée, de bromure.

3. Procédé selon la revendication 1 ou 2, dans lequel la conversion s'effectue avec un composé de formule 3, dans laquelle
R peut être un radical choisi dans le groupe constitué de méthoxy, éthoxy, propoxy, isopropoxy, isopropényloxy, butoxy, O-N-succinimide, O-N-phtalimide, phényloxy, nitrophényloxy, fluorophényloxy, pentafluorophényloxy, vinyloxy et 2-allyloxy.

4. Procédé selon la revendication 1, 2 ou 3, dans lequel la conversion s'effectue avec un composé de formule 2, dans laquelle
Y⁻ peut être hexafluorophosphate.

5. Procédé selon l'une des revendications 1 à 4, **caractérisé en ce que**
la conversion finale du composé de formule 4 en composé de formule 1 s'effectue à l'aide d'un sel CatX, dans laquelle Cat⁺ est choisi dans le groupe constitué de Li⁺, Na⁺, K⁺, Mg2+, Ca2+, de cations organiques comportant un N quaternaire (par ex. N,N-dialkylimidazolium, tétra-alkylammonium) et dans laquelle X⁻ peut avoir les significations mentionnées précédemment.

6. Composés de formule 2 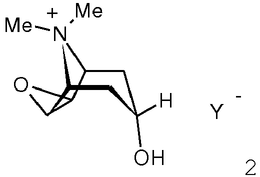 dans laquelle
Y⁻ est un anion lipophile à charge simplement négative choisi dans le groupe constitué de l'hexofluorophosphate, du tétrafluoroborate, et du tétraphénylborate.

7. Utilisation de composés de formule 2 selon la revendication 6 comme composés de départ pour la production de composé de formule 1.

8. Procédé de production de composés de formule 1, **caractérisé en ce qu'**un composé de formule 2 selon la revendication 6 est utilisé comme composé de départ pour la production de composés de formule 1.

9. Composés de formule 4 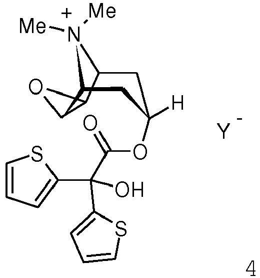 dans laquelle
Y⁻ est un anion lipophile à charge simplement négative choisi dans le groupe constitué de l'hexofluorophosphate, du tétrafluoroborate, et du tétraphénylborate.

10. Utilisation de composés de formule 4 selon la revendication 9 comme composés de départ pour la production de composés de formule 1.

11. Procédé de production de composés de formule 1, **caractérisé en ce qu'**un composé de formule 4 selon la revendication 9 est utilisé comme composé de départ pour la production de composés de formule 1.
